# EUROPEAN PATENT APPLICATION

(11) **EP 4 537 739 A1**
(43) Date of publication of application: **16.04.2025**
(21) Application number: 23202775.5
(22) Date of filing: 10.10.2023
(51) Int. Cl.: A61B 5/00, G16H 50/20

(54) **ONSET OF PAIN**

(71) Applicant: Koninklijke Philips N.V., 5656 AG Eindhoven (NL)
(72) Inventor: VAN DOOREN, Marieke, Eindhoven (NL); GERHARDT, Lutz Christian, Eindhoven (NL); JOHNSON, Mark Thomas, Eindhoven (NL); VAN LIESHOUT, Ron Martinus Laurentius, Eindhoven (NL)
(74) Representative: Philips Intellectual Property & Standards

(57) **Abstract**

Proposed concepts thus aim to provide schemes, solutions, concept, designs, methods and systems pertaining to indicating onset of pain in a subject. In particular, embodiments aim to provide a method for indicating onset of pain in a subject. This can be achieved by analysing a representation of subsurface tissue of the subject to detect one or more pain features indicative of onset of pain in the subject. From the one or more pain features, an indication of (past, present, or future) pain onset in the subject can be determined.

## Description

### FIELD OF THE INVENTION

This invention relates to the field of pain onset, and more specifically, to the field of indicating onset of pain.

### BACKGROUND OF THE INVENTION

Early pain onset detection via an objective measurement of sweat production and sweat gland/duct anatomy is relevant for all sorts of personal health solutions, such as Intense Pulsed Light (IPL), mechanical epilators, and shavers with heated blades, etc. Detection of early onset of pain can allow for quicker intervention or prevention.

To assess acute pain, clinics typically use questionnaires such as the Visual Analogue Scale (VAL) scoring system, or a range of faces in varying states of pain. Self-reports and questionnaires are a subjective measurement, however, and have a few difficulties. For instance, it is difficult to do continuous measures over time as most self-reports are discrete in time. Therefore, an objective measurement of early pain onset would have certain benefits over questionnaires.

A known objective measurement technique is electrodermal activity or Galvanic skin response which measures skin conductance in the upper skin layers (0.2mm) when sweat has reached the skin surface. This therefore leads to a latency between the pain response activating the sweat glands and the measurement of said response on the skin surface. This results in a delayed pain measurement. Also, due to reabsorption of electrolytes and water back into the interstitial tissue fluid, the skin-surface related pain measurement is non-linear and can be, especially in cases of low pain response, non-responsive. In other words, in some cases, the sweat and its properties needed to activate a pain measurement do not even reach the surface of the skin. Furthermore, this measurement requires electrodes. It is thus a key objective of the present invention to provide a faster and more sensitive image-based measurement of pain onset (or a precursor thereof).

### SUMMARY OF THE INVENTION

The invention is defined by the claims.

According to examples in accordance with an aspect of the invention, there is provided a method for indicating onset of pain in a subject.

The method comprises: analysing a representation of subsurface tissue of the subject to detect at least one pain feature indicative of onset of pain in the subject; and determining an indication of pain onset in the subject based on the at least one pain feature.

Proposed concepts thus aim to provide schemes, solutions, concepts, designs, methods and systems pertaining to indicating onset of pain in a subject. In particular, embodiments aim to provide a method for indicating onset of pain in a subject by analysing a representation of subsurface tissue of the subject to detect one or more pain features indicative of onset of pain in the subject. From the one or more pain features, an indication of (past, present, or future) pain onset in the subject can be determined.

In other words, it is proposed that by analysing a representation of subsurface tissue of a subject, pain features which are indicative of onset of pain in the subject (these may also be referred to as pain precursors) can be detected. For example, by analysing (imaging showing the anatomy, structure, geometry or physiology of) a sweat gland or sweat gland duct under the skin, an indication of pain onset in the subject can be determined. This is because in response to pain, the sweat glands of a subject activate, and therefore features of and around sweat glands can be used as indicators of pain onset.

By analysing a representation of subsurface tissue of a subject, an indication of pain onset can be determined quicker than by analysing a representation of surface tissue of a subject. This is because, for example, in the case of sweat glands, sweat is generated beneath the skin before it rises to the surface. By studying subsurface tissue, onset of pain can therefore be detected as soon as physiological changes occur, rather than waiting for the effects of these changes to reach the surface of the subject, e.g., for sweat to reach the skin surface.

In a simple example, a change in sweat gland anatomy can be detected in a microwave image, and an indication of pain onset in the subject can thus be rapidly determined, even perhaps before the subject feels any pain, i.e., the indication can indicate future pain onset. In other words, a change in sweat gland geometry or flow rate can be used to indicate future pain onset before the subject actually feels any pain. This determination can then potentially be used to automatically adjust a device or system which is being used on the subject, e.g., to reduce the subject's pain or prevent it.

In summary, the inventors have realised that by analysing subsurface tissue, rather than the more typical surface tissue, an indication of onset of pain can be determined more rapidly, and thus this information potentially acted on sooner. Furthermore, by using subsurface imaging, electrodes are not required to determine an indication of pain onset.

This invention may be of particular use in the field of personal healthcare devices which can lead to incidental pain, such as Intense Pulsed Light (IPL) devices, mechanical epilators, shavers with heated blades, and radiofrequency heating sources.

Ultimately, an improved method for indicating onset of pain in a subject is provided.

In some embodiments, the representation may comprise at least one of: two microwave images; two thermal images; two near-infrared spectra; two hyperspectral images; two time-domain near-infrared spectroscopy images or spectra; two photon arrival time histograms; two spectral polarization difference images; two light sheet microscopy images; two ultrasound images; and/or two dynamic optical coherence tomography images. These are all useful forms of representation from which pain features can be detected. Having at least two images (separated in time and potentially space) allows for changes between the images to be analysed in order to detect pain features. Further, if the timings of the images are accounted for, a rate of change can be determined in order to detect further pain features.

In some embodiments, the method may further comprise obtaining the representation of subsurface tissue of the subject. This allows the method to be more self-contained.

In some embodiments, obtaining the representation may comprise retrieving the representation from a data store. In this way, representations can be retrieved from a memory in order to determine indications of pain onset in the past, for example, to adjust future processes in order to reduce subject pain.

In some embodiments, the representation may comprise two microwave images, and obtaining the representation may comprise capturing two microwave images of subsurface tissue of the subject. Microwave images have been found to be an effective form of representation for detecting pain features.

In some embodiments, the representation may comprise two thermal camera images, and obtaining the representation may comprise capturing two thermal images of subsurface tissue of the subject. Thermal images have been found to be an effective form of representation for detecting pain features.

In some embodiments, the representation may comprise two near-infrared spectra or images, and obtaining the representation may comprise capturing two near-infrared spectra or images of subsurface tissue of the subject. Near-infrared spectra or images have been found to be an effective form of representation for detecting pain features.

In some embodiments, the represented subsurface tissue of the subject may comprise at least one of a sweat gland; a sweat gland duct; and/or a secretory coil of a sweat gland. These are subsurface structures which are beneficial to be represented for the detection of pain features.

In some embodiments, determining an indication of pain onset may comprise comparing the at least one pain feature to at least one reference pain feature; and determining an indication of pain onset based on the comparison. This allows for pain features to be checked against predetermined reference features, which could, for example, represent no onset of pain or an onset of pain. If the reference pain feature represents no onset of pain, then if the determined pain feature deviates by more than a certain predetermined threshold (absolutely or relatively) from the reference pain feature, then an indication of pain onset can be determined. If the reference pain feature represents onset of pain, then if the determined pain feature matches the reference pain feature within a certain predetermined threshold (absolutely or relatively), then an indication of pain onset can be determined. The reference pain feature may therefore be a generic reference pain feature applied regardless of the subject.

In some embodiments, the at least one reference pain feature may comprise at least one personal reference pain feature, and the method may further comprise: providing a stimulus to a tissue surface of the subject; obtaining a reference representation of subsurface tissue of the subject beneath the stimulated tissue surface; and analysing the reference representation to determine at least one personal pain feature for the subject. By providing a stimulus to a tissue surface of the subject before (or essentially simultaneously with) capturing a reference representation of the subsurface tissue beneath the stimulation, a personalised reference pain feature can be obtained which represents onset of pain. Future determined pain features can then be compared to this personalized reference pain feature, and if they match within a certain predetermined threshold (absolutely or relatively), an indication of pain onset can be determined. In this way, the personalised pain responses of subjects can be accounted for.

In some embodiments, the at least one pain feature may comprise at least one of a change and/or rate of change of: sweat gland geometry such as the diameter, size, length, and/or volume of the sweat gland and/or sweat gland duct; sweat gland lumen dilation; sweat flow; sweat gland secretory coil diameter and/or volume; magnitude of absorption of a specific wavelength; photon arrival time; light intensity and/or contrast in the entirety of and/or portions of the representation; and/or temperature in the entirety of and/or portions of the representation. These are all features which can effectively indicate onset of pain in a subject and be efficiently detected. The skilled person would understand which features can or cannot be detected depending on the forms of representations available. The change or rate of change can be absolute or relative.

In some embodiments, the representation may consist of at least one of: a microwave image; a thermal image; a near-infrared spectrum; a hyperspectral image; a time-domain near-infrared spectroscopy image or spectrum; a photon arrival time histogram; a spectral polarization difference image; a light sheet microscopy image; an ultrasound image; and/or a dynamic optical coherence tomography image. Having only one image allows for pain features to be more efficiently detected, for instance, absolute features such as an absolute length, diameter, size, and/or volume, of a sweat gland, sweat gland duct, and/or secretory coil etc.

In some embodiments, the method may further comprise generating a control signal for controlling a system and/or device responsive to determining an indication of pain onset in the subject and based on the at least one pain feature. This allows the operation of a system and/or device to be adjusted to potentially reduce pain in the subject, for example, if the system and/or device was the source of said pain onset. The specific adjustment can be based on the pain feature, e.g., to make a large adjustment in response to a pain feature indicating a lot of pain, and to only make a small adjustment in response to a pain feature indicating a small amount of pain.

In some embodiments, obtaining a reference representation of subsurface tissue of the subject beneath the stimulated tissue surface may further comprise obtaining a corresponding reference time for correlating the stimulus to the reference representation of subsurface tissue. This allows for time-correlation of the stimulus to the reference representation and for a more specific analysis of acute pain (e.g., differentiation from increased sweating due to arousal or emotional stress).

According to another aspect of the invention, there is provided a computer program comprising code means for implementing the method of any herein disclosed method when said program is run on a processing system.

According to another aspect of the invention, there is provided a system for indicating onset of pain in a subject. The system comprising a processor arrangement configured to: analyse a representation of subsurface tissue of the subject to detect at least one pain feature indicative of onset of pain in the subject; and determine an indication of pain onset in the subject based on the at least one pain feature.

In some embodiments, the system may further comprise an imaging device for capturing the representation. This allows the system to be more self-contained.

According to another aspect of the invention, there is provided a personal health care device comprising the system of any herein disclosed embodiment.

In some embodiments, the personal health care device may comprise an Intense Pulsed Light (IPL) device, a mechanical epilator, a shaver with a heated blade, or a radiofrequency heating source. These are all personal health care devices which may cause pain in a subject, and the settings of which can potentially be adjusted in response to a determined indication of pain onset.

Thus, there may be proposed concepts for indicating onset of pain in a subject, and this may be done based on analysing a representation of subsurface tissue of a subject to detect at least one pain feature indicative of onset of pain in the subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for indicating onset of pain in a subject according to a proposed embodiment;
Fig. 2 is a diagram showing the simplified anatomy of a sweat gland;
Fig. 3A is a diagram showing an example galvanic skin response to pain as a function of time after a pain stimulus is applied;
Fig. 3B is a diagram showing an example pain feature response to pain as a function of time after a pain stimulus is applied;
Fig. 4 is a flow diagram of a method for indicating onset of pain in a subject according to a proposed embodiment;
Figs. 5A-5D are flow diagrams of methods for indicating onset of pain in a subject according to proposed embodiments respectively;
Fig. 6 is a flow diagram of a method for indicating onset of pain in a subject according to a proposed embodiment;
Figs. 7A and 7B are block diagrams of systems for indicating onset of pain in a subject according to proposed embodiments respectively; and
Fig. 8 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to indicating onset of pain in a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for indicating onset of pain in a subject. This can be achieved by analysing (from subsurface images) a representation of subsurface tissue of the subject to detect one or more pain features indicative of onset of pain in the subject. From the one or more pain features, an indication of (past, present, or future) pain onset in the subject can be determined.

Proposed concepts thus aim to provide schemes, solutions, concepts, designs, methods and systems pertaining to indicating onset of pain in a subject.

In other words, it is proposed that by analysing a representation of subsurface tissue of a subject, pain features which are indicative of onset of pain in the subject can be detected. For example, by analysing a sweat gland or sweat gland duct under the skin, an indication of pain onset in the subject can be determined. This is because in response to pain, the sweat glands of a subject activate, and therefore features of and around sweat glands can be used as indicators of pain onset.

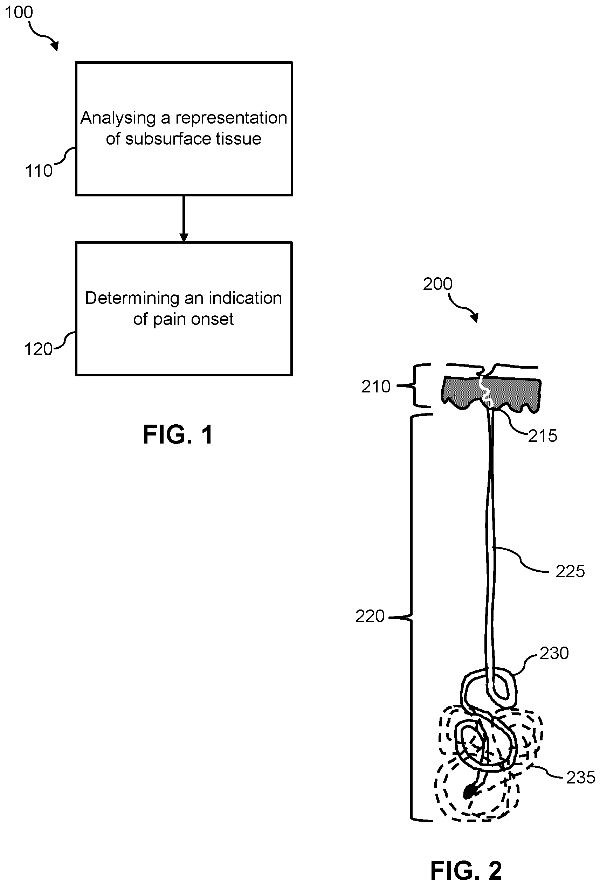

Referring now to Fig. 1, there is depicted a simplified flow diagram of a method 100 for indicating onset of pain in a subject according to a proposed embodiment.

The method 100 begins with the step 110 of analysing a representation of subsurface tissue of the subject to detect at least one pain feature indicative of onset of pain in the subject, i.e., in a subsurface image. A representation of subsurface tissue can be understood as a direct representation of subsurface tissue. In other words, a representation of subsurface tissue does not comprise a representation of surface tissue from which representations of subsurface tissues can be derived.

In this embodiment, the representation comprises at least one of: two microwave images; two thermal images; two near-infrared spectra; two hyperspectral images; two time-domain near-infrared spectroscopy images or spectra; two photon arrival time histograms; two spectral polarization difference images; two light sheet microscopy images; two ultrasound images; and/or two dynamic optical coherence tomography images. These are all useful forms of representation from which pain features can be detected. Having at least two images (separated in time and potentially space) allows for changes between the images to be analysed in order to detect pain features. Further, if the timings of the images are accounted for, a rate of change can be determined in order to detect further pain features. In some embodiments, the two time-domain near-infrared spectroscopy images or spectra can be obtained/captured with a single photon avalanche diode, i.e., a SPAD sensor. The skilled person would understand, however, that in other embodiments any suitable subsurface imaging technique can be used.

In yet other embodiments, the representation may consist of at least one of: a microwave image; a thermal image; a near-infrared spectrum; a hyperspectral image; a time-domain near-infrared spectroscopy image or spectrum; a photon arrival time histogram; a spectral polarization difference image; a light sheet microscopy image; an ultrasound image; and/or a dynamic optical coherence tomography image. Having only one image allows for pain features to be more efficiently detected, for instance, absolute features such as an absolute length, diameter, size, and/or volume, of a sweat gland, sweat gland duct, and/or secretory coil etc.

In this embodiment, the represented subsurface tissue of the subject comprises at least one of: a sweat gland; a sweat gland duct; and/or a secretory coil of a sweat gland. These are subsurface structures which are beneficial to be represented for the detection of pain features.

A pain feature can essentially be understood as a proxy parameter indicative of pain onset in a subject.

In step 120, an indication of pain onset in the subject is determined based on the at least one pain feature. An indication of pain onset can be an indication of pain onset in the past, present, or future. In other words, indicating pain onset can be understood as determining or predicting pain onset, i.e., indicating a point in time when pain is onset or pain reaches a certain level. In yet other words, onset of pain in a subject refers to the point in time when a subject first begins to experience pain, and thus an indication of pain onset is an indication of the point in time (which may be past, present, or (near) future) when a subject first begins (or is about) to experience pain.

In this embodiment, the at least one pain feature comprises at least one of a change and/or rate of change of: sweat gland geometry such as the diameter, size, length, and/or volume of the sweat gland and/or sweat gland duct; (dynamic) sweat gland lumen dilation; sweat flow; sweat gland secretory coil diameter and/or volume; magnitude of absorption of a specific wavelength; photon arrival time; light intensity and/or contrast in the entirety of and/or portions of the representation; and/or temperature in the entirety of and/or portions of the representation. These are all features which can effectively indicate onset of pain in a subject and be efficiently detected. The skilled person would understand which features can or cannot be detected depending on the forms of representation available. The change or rate of change can be absolute or relative. For example, if the representation comprises two or more images, then the (absolute or relative) change or rate of change of each provided feature can be detected, whereas if the representation only comprises a single image, then the at least one pain feature can comprise an absolute measurement of at least one of the provided features.

In some embodiments, the method may further comprise generating a control signal for controlling a system and/or device responsive to determining an indication of pain onset in the subject and based on the at least one pain feature. This allows the operation of a system and/or device to be adjusted to potentially reduce pain in the subject, for example, if the system and/or device was the source of said pain onset. For example, parameters of a personal health care device (being used on the subject) can be adjusted based on the indication of pain onset and the at least one pain feature in order to try to reduce the subject's pain. For example, the control signal can be configured to adapt a waveform or intensity of a flash pulse for an IPL, adjusting a waveform of a pulse for epilators, or applying a temperature cooling effect, for instance, for heated blades.

Referring now to Fig. 2, there is depicted a diagram showing the simplified anatomy of a sweat gland 200. Layer 210 is the epidermis skin layer, and layer 220 is the dermis layer. There can be seen the acrosyringium (intraepidermal spiral duct) 215, the sweat gland duct 225 leading into the coiled duct 230 leading into the secretory coil 235. Sweat is produced in the secretory coil 235 in response to connected nerves which trigger changes in the sweat gland 200 anatomy/function upon stimulation. Once produced, the sweat then travels through the coiled duct 230, up the sweat gland duct 225, and then through the acrosyringium 215 to the skin surface.

For the representation of the subsurface tissue, it is thus preferable to make use of certain wavelengths of light (e.g., the electromagnetic spectrum including microwaves) which can image about 1-5mm beneath the surface of the skin, where sweat production originates in the secretory coil 235. In this way, faster and more sensitive assessment of pain onset can be performed compared to current methods in the art.

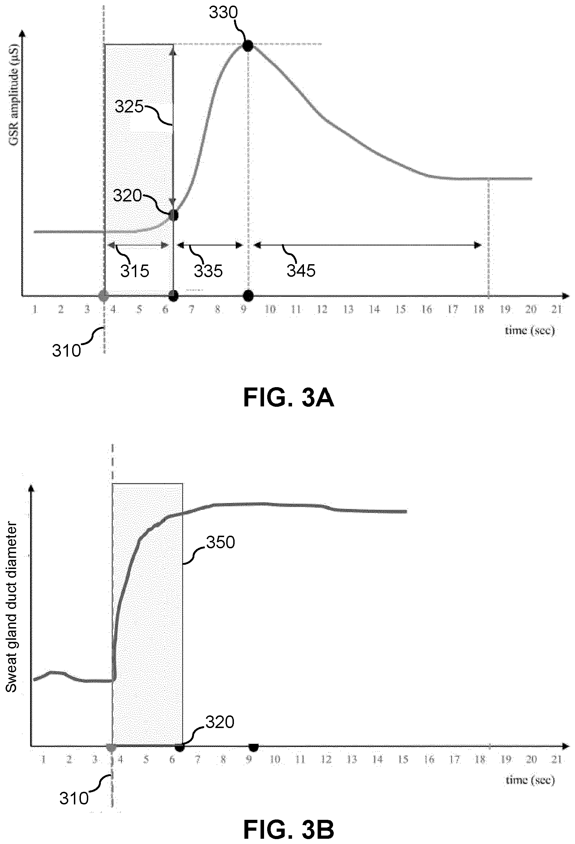

Referring now to Fig. 3A there is depicted a diagram showing an example galvanic skin response (GSR) to pain as a function of time after a pain stimulus is applied. In other words, the diagram shows an example GSR response signal (as a proxy parameter for acute pain onset) in response to an experienced stimulus, e.g., mechanical, optical, thermal, etc. The y-axis shows the GSR amplitude in units of microsiemens, and the x-axis shows time in units of seconds. Stimulus occurs at time 310, and there is then a latency 315 before a measurable onset of pain at point 320 (using GSR). GSR amplitude then increases by amplitude 325 during rise time 335 until peak GSR amplitude 330 is reached before a gradual fall over recovery time duration 345. It can therefore clearly be seen that, using GSR, there is a latency 315 between activation of the sweat glands (i.e., at time 310 synchronously with the stimulus) and the measurability of said response on the skin surface. This latency 315 is roughly two seconds.

For instance, pain assessment using GSR is only a measurement of skin conductance in the upper skin layers (0.2 mm) when sweat has reached the skin surface. Skin conductance typically shows a latency of 2 seconds. The sweat gland where the sweat drops originate is situated 1-5 mm below the skin surface. Therefore, the current proposal measures deeper in the skin where the sweat gland is situated. This results in faster and more reliably sensitive detection of pain since the sweat measurement does not require the sweat to have reached the skin surface.

In other words, very early detection of pain onset (and therefore pain prevention) are not possible with known objective pain measurement solutions. Current pain measurements only work by utilizing the sweat properties on the skin surface, either by galvanic skin response (GSR) or optical surface measurement. This has two major disadvantages. One, that there is a latency between the pain response activating the sweat glands and the measurement of said response on the skin surface resulting in a delayed pain measurement. Two, that due to reabsorption of electrolytes and water back into the interstitial tissue fluid, the skin-surface related pain measurement is non-linear and can be, especially in cases of low pain response, non-responsive. In other words, the sweat and its properties needed to activate the skin-surface measurement might not even reach the surface of the skin.

For example, whilst it is known to optically detect sweat droplets on the surface of the skin, this has the drawback that the sweat does not always reach the skin surface due to sweat reabsorption along the sweat duct when sweat moves towards the skin surface. As this happens, some pain signals will be missed if only surface imaging is used. Therefore, full prevention of pain may not even be possible due to sweat reabsorption along the sweat duct when sweat moves towards the skin surface therefore resulting in pain not being detected. Reabsorption is a biochemical process and is always present (at both pain events and regular thermoregulatory sweating).

Referring now to Fig. 3B, there is depicted a diagram showing an example pain feature response to pain as a function of time after a pain stimulus is applied in this case, sweat gland duct diameter. In contrast to the galvanic skin response, the pain feature begins measurably changing almost immediately at the time of stimulus 310. The benefit in time difference of measurability is therefore given by the box 350, wherein point 320 represents the point of measurability of pain onset if using GSR. This benefit is not restricted only to measuring sweat gland duct diameter, but may be seen with any other herein disclosed pain features.

Therefore, in addition to solving the problem of faster and more complete detection of pain onset, the present invention also solves the problem of having to attach electrodes to the skin (and applying conductive gel to increase the signal quality). For example, a camera of a personal health care device could be used as a pain sensor (i.e., an imaging device to capture the representation and a processing arrangement to process the captured representation). In this way, no direct skin contact is required for indicating pain onset, i.e., determining or predicting, whilst also simplifying the system architecture. In other words, typical electrical surface pain measurements are replaced with optical, electromagnetic, or even (photo-)acoustic subsurface (image-based) pain measurements, potentially while reusing existing imaging systems (optical, electromagnetic, ultrasound acoustic, etc.) in a personal health care device.

In embodiments of the proposed method (e.g. method 100 of Fig. 1), wherein the representation comprises two images/spectra, these images/spectra should preferably be captured at points 310 and 320 in order to more easily detect a (maximal) change or rate of change in a pain feature. In embodiments in which the representation comprises three or more images/spectra, these images/spectra should preferably be captured between points 310 and 320 in order that a more informative rate of change of a pain feature can be detected. In embodiments in which the representation consists of only a single image/spectrum, the image/spectrum should preferably be captured any time immediately after point 310 and before point 320.

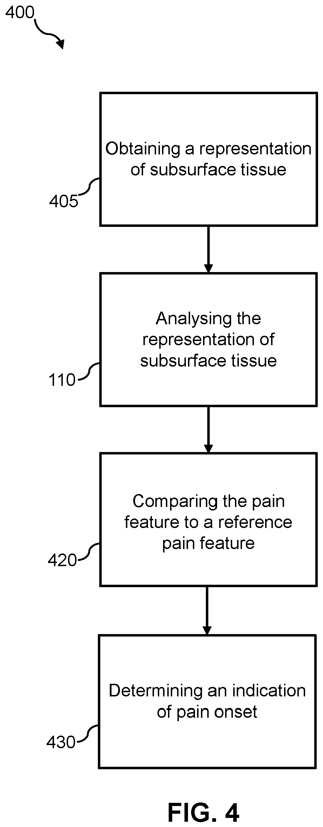

Referring now to Fig. 4, there is depicted a flow diagram of a method 400 for indicating onset of pain in a subject according to a proposed embodiment. Step 110 is substantially the same as has been described in relation to method 100 of Fig. 1.

The method 400 begins with step 405 of obtaining a representation of subsurface tissue of the subject. This allows the method to be more self-contained.

Step 420 comprises comparing the at least one pain feature to at least one reference pain feature. Step 430 comprises determining an indication of pain onset based on the comparison. This allows for pain features to be checked against predetermined reference features, which could, for example, represent no onset of pain or an onset of pain. If the reference pain feature represents no onset of pain, then if the determined pain feature deviates by more than a certain predetermined threshold (absolutely or relatively) from the reference pain feature, then an indication of pain onset can be determined. If the reference pain feature represents onset of pain, then if the determined pain feature matches the reference pain feature within a certain predetermined threshold (absolutely or relatively), then an indication of pain onset can be determined. The reference pain feature may therefore be a generic reference pain feature applied regardless of the subject.

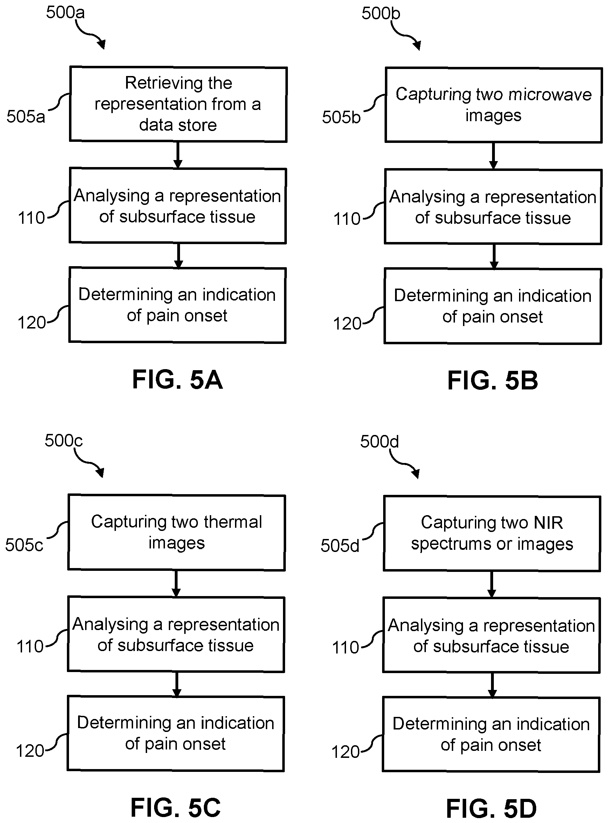

Referring now to Figs. 5A-5D are flow diagrams of methods, 500a, 500b, 500c, 500d, for indicating onset of pain in a subject according to proposed embodiments respectively. Steps 110 and 120 are much the same as have been described in relation to method 100 of Fig. 1.

Step 505a of method 500a comprises retrieving the representation from a data store. In this way, representations can be retrieved from a memory in order to determine indications of pain onset in the past, for example, to adjust future processes in order to reduce subject pain.

Step 505b of method 500b comprises capturing two microwave images of subsurface tissue of the subject. In this embodiment, the representation comprises two microwave images. Microwave images have been found to be an effective form of representation for detecting pain features, such as, for example, a change (and/or rate of change of): sweat gland geometry such as the diameter, size, length, and/or volume of the sweat gland and/or sweat gland duct; sweat gland lumen dilation; sweat flow; sweat gland secretory coil diameter and/or volume; and/or light intensity and/or contrast in the entirety of and/or portions of the microwave images.

For example, microwave imaging can be used to detect filled/unfilled ducts and deep glands in subsurface tissue. In terms of microwave frequencies, filled and unfilled ducts absorb the microwave light differently. The dimensions of ducts are typically around several millimetres in length and around 0.1-1mm in diameter. These are typical microwave wavelengths.

An example of a wideband, multispectral microwave camera is, for example, the MIT microwave camera. When this camera takes each measurement, the microwave emitter sweeps through a frequency range of 7.835 GHz to 12.817 GHz over 10ms. Of course, different materials respond to the microwaves differently at lower and higher frequencies, and the camera can separate out these spectra. This can provide an image with multiple frequency response 'colours', and the patterns of colours can thus provide information about the material. Of course, in other embodiments, any suitable microwave camera can be used.

Due to their dimensions, the ducts and glands will act as microwave antennas. Microwaves will resonate in these structures in a manner analogous to acoustic waves in an organ pipe. In the absence of sweat production, the sweat gland is essentially collapsed and contains no fluid. As sweat production initiates, sweat starts to flow up the duct, from the sweat gland towards the skin surface. As a consequence, a new fluid structure emerges with a length which increases rapidly up to several millimetres.

The fluid and the tissue will show different microwave impedances. Specifically, sweat has roughly two times the complex permittivity as the dermis and epidermis (the upper layers of the skin where the sweat ducts are found). As a consequence, the microwave camera will detect the presence of a new feature, i.e., the sweat, in the dermis/epidermis.

A characteristic of the new feature is that its length will rapidly increase (to eventually fill the duct) in the course of around one second. As the length increases, the wavelength of the most absorbed microwaves will increase accordingly. Hence, the characteristic feature for (early) sweat production is the appearance of a new feature wherein the wavelength of microwave absorption increases during a period of around 1 second towards the length of the duct (around 5mm).

As, typically, all sweat glands in the microwave image will respond in a similar manner at precisely the same moment of time, the image is likely to show a multiplicity of such signals (actually, an array of such new absorbers) which will amplify the characteristic signal. In such a way, both the sensitivity and the specificity of the response will increase from the case where only a single sweat gland is being imaged.

Step 505c of method 500c comprises capturing two thermal images of subsurface tissue of the subject. In this embodiment, the representation comprises two thermal camera images. Thermal images have been found to be an effective form of representation for detecting pain features, such as, for example, a change (and/or rate of change of): sweat gland geometry such as the diameter, size, length, and/or volume of the sweat gland and/or sweat gland duct; sweat gland lumen dilation; sweat flow; sweat gland secretory coil diameter and/or volume; temperature in the entirety of and/or portions of the representation; and/or light intensity and/or contrast in the entirety of and/or portions of the thermal camera images.

For example, once a sweat drop originates, a local cooling (temperature drop) of the surrounding tissue can be detected. In other words, with thermal images, a characteristic image-derived feature (pain feature) indicative of acute pain onset after the stimulus is the rapid appearance of many `colder' spots, i.e., thermal spots lower than body temperature indicating sweat production (or sweat excretion on the skin surface).

Step 505d of method 500d comprises capturing two near-infrared spectra or images of subsurface tissue of the subject. In this embodiment, the representation comprises two near-infrared spectra or images of subsurface tissue of the subject. Near-infrared spectra or (multispectral or hyperspectral) images have been found to be an effective form of representation for detecting pain features, such as, for example, a change (and/or rate of change of): sweat gland geometry such as the diameter, size, length, and/or volume of the sweat gland and/or sweat gland duct; sweat gland lumen dilation; sweat flow; sweat gland secretory coil diameter and/or volume; magnitude of absorption of a specific wavelength; and/or light intensity and/or contrast in the entirety of and/or portions of the thermal camera images.

For instance, near-infrared spectralimages may comprise time-domain near-infrared spectralimages captured with a single photon avalanche diode (SPAD) sensor. In this embodiment, a characteristic feature (pain feature) is the rapid expansion of the sweat gland or secretory coil (diameter change) detected from the images/spectra. For example, the dynamic dilution of the gland lumen or duct can be studied. In some embodiments, this can be done by using a shift in a photon arrival time histogram (i.e., measuring the shift of the peak between two histograms separated in time) as a characteristic feature (pain feature) for sweat production stimulated through a pain stimulus.

In other embodiments, spectral polarization different imaging (SPDI) can be used, i.e., a polarimetric optical "drill" dynamically accessing different sub-tissue depths. The diameter of the secretory lumen (i.e., the lumen of the secretory coil) can vary, depending on whether the gland is actively producing sweat fluid. In these embodiments, a characteristic feature (pain feature) is the rapid expansion of the secretory coil (diameter change) detected in images acquired using a polarimetric optical drill.

In other embodiments, light sheet microscopy imaging can be used. Light sheet microscopy is an imaging technique that uses a thin sheet of laser light to selectively illuminate a specific plane (or depth) within a biological sample, reducing photodamage and allowing for rapid 3D imaging. In these embodiments, a localised high contrast light beam can be used at specific tissue depths, e.g., at one or two depths for differential measurement. The main sweat duct or the secretory coil can be imaged and the duct/coil expansion can be used as a characteristic feature (pain feature) for sweat production indicative of pain.

Furthermore, in some embodiments, an image contrast or brightness profile can be detected and used as an indicator and proxy parameter (pain feature). Sweat is transparent, while tissue and the gland appear dark. A change in image contrast or intensity (e.g., a step function from dark to bright to dark along an axis, e.g., indicating an open sweat duct) can therefore be used to indicate onset of pain in the subject.

In other embodiments, dynamic optical coherence tomography (OCT) can be used. In OCT images, eccrine sweat glands in the stratum corneum of the epidermis are clearly recognizable as spiral lumens with a high reflection light intensity due to the large difference in refractive index at the wall of the spiral lumen between sweat and the keratinous epidermal tissue.

The reflected light intensity in active sweat glands suddenly increase in response to sound stress (i.e., a stress response in response to certain sounds). Therefore, a rapid increase in light reflection could be used as a characteristic feature (pain feature) for pain-induced sweat production after a stimulus.

To distinguish between pain-induced and heat-induced/emotional stress-induced sweat response, in some embodiments, sweat generation imaging per individual sweat gland or type of gland (eccrine and apocrine) can be performed. Further, a ground truth pain measurement/GSR can be obtained and used to train a machine-learning model on images accordingly, i.e., used to annotate images including pain features for training.

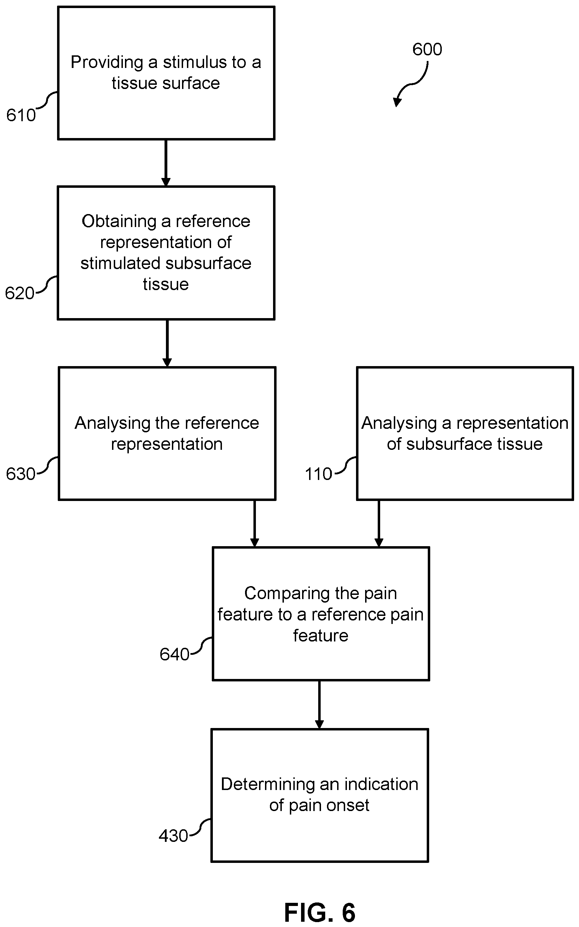

Referring now to Fig. 6, there is depicted a flow diagram of a method 600 for indicating onset of pain in a subject according to a proposed embodiment. Steps 110 and 430 are substantially the same as have been described in relation to methods 100 and 400 respectively.

Step 610 comprises providing a stimulus to a tissue surface of the subject. The stimulus may comprise a thermal, optical, mechanical, physical and/or chemical stimulus configured to induce pain onset in the subject. For example, in some embodiments, a mechanical and/or thermal actuator can be used to deliver a well-defined stimulus to a tissue surface of the subject.

Step 620 comprises obtaining a reference representation of subsurface tissue of the subject beneath the stimulated tissue surface. In some embodiments, the reference representation comprises the same types of images/spectra as the representation (of step 110).

In some embodiments, step 620 also comprises obtaining a corresponding reference time for correlating the stimulus to the reference representation of subsurface tissue. This allows the reference representation to be time-correlated with the stimulus.

Step 630 comprises analysing the reference representation to determine at least one personal pain feature for the subject. Step 630 may be substantially the same as step 110 described in relation to method 100.

By providing a stimulus to a tissue surface of the subject before (or essentially simultaneously with) obtaining a reference representation of the subsurface tissue beneath the stimulation, a personalised reference pain feature can be obtained which represents onset of pain. Future determined pain features can then be compared to this personalized reference pain feature, and if they match within a certain predetermined threshold (absolutely or relatively), an indication of pain onset can be determined. In this way, the personalised pain responses of subjects can be accounted for.

Step 640 comprises comparing the at least one pain feature to the at least one personal reference pain feature.

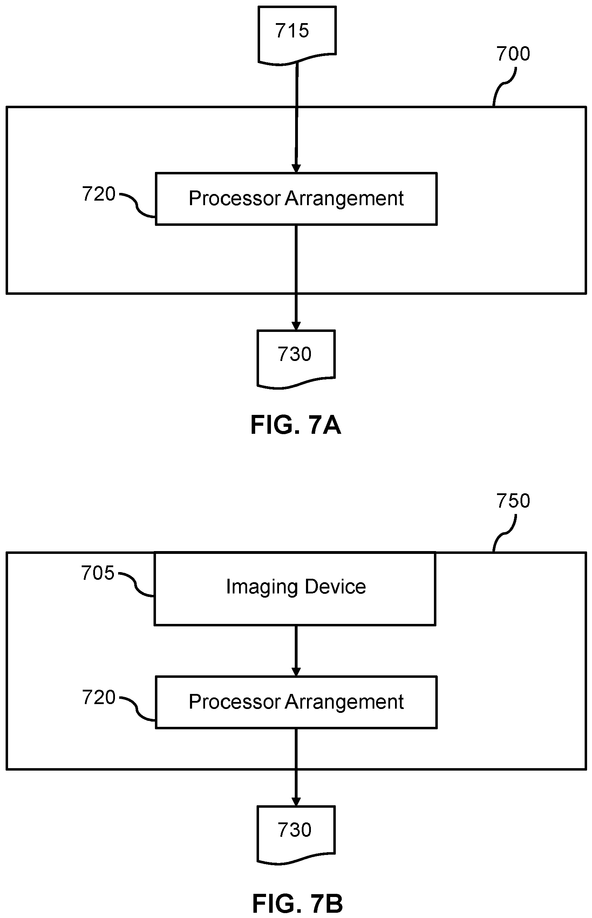

Referring now to Fig. 7A, there is depicted a system 700 for indicating onset of pain in a subject according to a proposed embodiment. The system 700 comprises a processor arrangement 720.

The system 700 is configured to indicate onset of pain in a subject by processing inputs 715. The inputs 715 comprise a representation of subsurface tissue of the subject. The processor arrangement 720 is then configured to analyse these inputs 715 to detect at least one pain feature indicative of pain in the subject. The output 730 comprises an indication of pain onset in the subject based on the at least one pain feature.

Referring now to Fig. 7B, there is depicted a system 750 for indicating onset of pain in a subject according to a proposed embodiment. The system 750 comprises the system 700 of Fig. 7A and an imaging device 705 for capturing the representation, i.e., for generating the inputs 715.

In some embodiments, there is provided a personal health care device comprising the system of any herein disclosed embodiment, e.g., system 700 or system 750. In some embodiments, the personal health care device can comprise an Intense Pulsed Light (IPL) device, a mechanical epilator, a shaver with a heated blade, or a radiofrequency heating source. These are all personal health care devices which may cause pain in a subject, and the settings of which can potentially be adjusted in response to a determined indication of pain onset. Many personal health care devices already comprise imaging devices of one form or another, and these can thus be repurposed to capture the representation, thereby keeping the device structurally efficient.

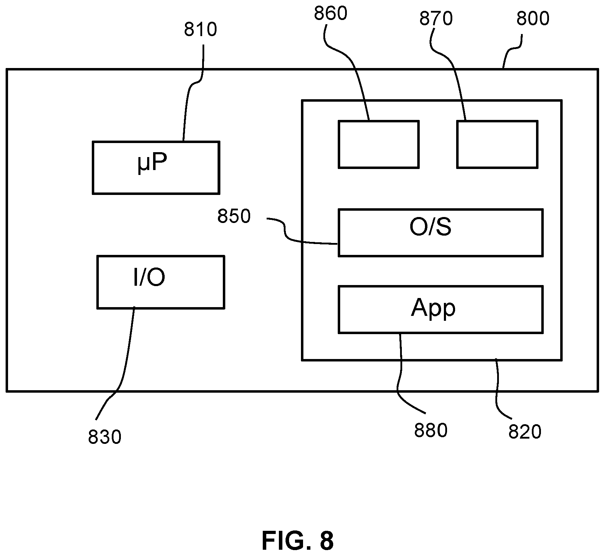

Fig. 8 illustrates an example of a computer 800 within which one or more parts of an embodiment may be employed. Various operations discussed above may utilize the capabilities of the computer 800. In this regard, it is to be understood that system functional blocks can run on a single computer or may be distributed over several computers and locations (e.g. connected via internet).

The computer 800 includes, but is not limited to, PCs, workstations, laptops, PDAs, palm devices, servers, storages, and the like. Generally, in terms of hardware architecture, the computer 800 may include one or more processors 810, memory 820 and one or more I/O devices 830 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 860, source code 870, and one or more applications 880 in accordance with exemplary embodiments. As illustrated, the application 880 comprises numerous functional components for implementing the features and operations of the exemplary embodiments. The application 880 of the computer 800 may represent various applications, computational units, logic, functional units, processes, operations, virtual entities, and/or modules in accordance with exemplary embodiments, but the application 880 is not meant to be a limitation.

The operating system 850 controls the execution of other computer programs, and provides scheduling, input-output control, file and data management, memory management, and communication control and related services. It is contemplated by the inventors that the application 880 for implementing exemplary embodiments may be applicable on all commercially available operating systems.

Application 880 may be a source program, executable program (object code), script, or any other entity comprising a set of instructions to be performed. When a source program, then the program is usually translated via a compiler (such as the compiler 860), assembler, interpreter, or the like, which may or may not be included within the memory 820, so as to operate properly in connection with the O/S 850. Furthermore, the application 880 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 830 may include input devices such as, for example but not limited to, a mouse, keyboard, scanner, microphone, camera, etc. Furthermore, the I/O devices 830 may also include output devices, for example but not limited to a printer, display, etc. Finally, the I/O devices 830 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 830 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at start-up, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 880 and the O/S 850 are read, in whole or in part, by the processor 810, perhaps buffered within the processor 810, and then executed.

When the application 880 is implemented in software it should be noted that the application 880 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 880 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1, 4, 5A-5D, and 6 and the systems of Fig. 7A and 7B, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 8 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method for indicating onset of pain in a subject, the method comprising:
analysing a representation of subsurface tissue of the subject (110) to detect at least one pain feature indicative of onset of pain in the subject; and
determining an indication of pain onset in the subject (120) based on the at least one pain feature.

2. The computer-implemented method of claim 1, wherein the representation comprises at least one of: two microwave images; two thermal images; two near-infrared spectrum; two hyperspectral images; two time-domain near-infrared spectroscopy images or spectra; two photon arrival time histograms; two spectral polarization difference images; two light sheet microscopy images; two ultrasound images; and/or two dynamic optical coherence tomography images.

3. The computer-implemented method of claim 1 or 2, further comprising:
obtaining the representation of subsurface tissue of the subject (405).

4. The computer-implemented method of claim 3, wherein obtaining the representation comprises:
retrieving the representation from a data store (505a).

5. The computer-implemented method of claim 3, wherein the representation comprises two microwave images, and obtaining the representation comprises:
capturing two microwave images of subsurface tissue of the subject (505b).

6. The computer-implemented method of claim 3, wherein the representation comprises two thermal camera images, and obtaining the representation comprises:
capturing two thermal images of subsurface tissue of the subject (505c).

7. The computer-implemented method of claim 3, wherein the representation comprises two near-infrared spectra or images, and obtaining the representation comprises:
capturing two near-infrared spectra or images of subsurface tissue of the subject (505d).

8. The computer-implemented method of any prior claim, wherein the represented subsurface tissue of the subject comprises at least one of: a sweat gland; a sweat gland duct; and/or a secretory coil of a sweat gland.

9. The computer-implemented method of any prior claim, wherein determining an indication of pain onset comprises comparing the at least one pain feature to at least one reference pain feature (420); and determining an indication of pain onset based on the comparison (430).

10. The computer-implemented method of claim 9, wherein the at least one reference pain feature comprises at least one personal reference pain feature, and the method further comprises:
providing a stimulus to a tissue surface of the subject (610);
obtaining a reference representation of subsurface tissue of the subject beneath the stimulated tissue surface (620); and
analysing the reference representation (630) to determine at least one personal reference pain feature for the subject.

11. The computer-implemented method of any prior claim, wherein the at least one pain feature comprises at least one of a change and/or rate of change of: sweat gland geometry such as the diameter, size, length, and/or volume of the sweat gland and/or sweat gland duct; sweat gland lumen dilation; sweat flow; sweat gland secretory coil diameter and/or volume; magnitude of absorption of a specific wavelength; shift or change in a photon arrival time histogram; light intensity and/or contrast in the entirety of and/or portions of the representation; and/or temperature in the entirety of and/or portions of the representation.

12. A computer program comprising code means for implementing the method of any preceding claim when said program is run on a processing system.

13. A system (700) for indicating onset of pain in a subject, the system comprising:
a processor arrangement (720) configured to:
analyse a representation of subsurface tissue of the subject (715) to detect at least one pain feature indicative of onset of pain in the subject; and
determine an indication of pain onset in the subject (730) based on the at least one pain feature.

14. The system of claim 13, further comprising an imaging device (705) for capturing the representation.

15. A personal health care device comprising the system of claim 13 or 14, and preferably wherein the personal health care device comprises an Intense Pulsed Light device, a mechanical epilator, a shaver with a heated blade, or a radiofrequency heating source.
